# EUROPEAN PATENT APPLICATION

(11) **EP 4 435 720 A1**
(43) Date of publication of application: **25.09.2024**
(21) Application number: 23787558.8
(22) Date of filing: 04.04.2023
(51) Int. Cl.: G06T 7/70

(54) **IMAGE PROCESSING METHOD AND RELATED DEVICE**

(30) Priority: 13.04.2022 CN 202210384608
(71) Applicant: Huawei Technologies Co., Ltd., Shenzhen, Guangdong 518129 (CN)
(72) Inventor: CHEN, Wei, Shenzhen, Guangdong 518129 (CN); LU, Liangbing, Shenzhen, Guangdong 518129 (CN); ZHANG, Peng, Shenzhen, Guangdong 518129 (CN); GUO, Jingjing, Shenzhen, Guangdong 518129 (CN); WEI, Wei, Shenzhen, Guangdong 518129 (CN)
(74) Representative: Isarpatent
(86) International application number: PCT/CN2023/086213
(87) International publication number: WO 2023/197913

(57) **Abstract**

Embodiments of this application provide an image processing method. The method is applied to an electronic device. The electronic device is configured to obtain a plurality of frames of images of a front angle of view of a user. The method includes: determining a first route if a crosswalk is detected in the plurality of frames of images, where the first route is a safe walking route on the crosswalk; obtaining, based on the plurality of frames of images, a second route that the user actually walks; and outputting first indication information to the user if an included angle between the first route and the second route in a same reference system is greater than or equal to a preset angle, where the first indication information indicates that the second route is abnormal. In this way, when the user deviates from the crosswalk in a movement process, the first indication information is output to improve safety of the user when the user passes through the crosswalk.

## Description

This application claims priority to Chinese Patent Application No. 202210384608.3, filed with the China National Intellectual Property Administration on April 13, 2022 and entitled "IMAGE PROCESSING METHOD AND RELATED DEVICE", which is incorporated herein by reference in its entirety.

### TECHNICAL FIELD

Embodiments of this application relate to the field of terminal technologies, and in particular, to an image processing method and a related device.

### BACKGROUND

What visually impaired people want most is to live a normal daily life independently like ordinary people. Traveling is an important part of their lives.

Currently, the visually impaired people mainly rely on a blind cane during traveling to recognize an obstacle on a road. Because the blind cane is mainly used for front obstacles, in a scenario like a crosswalk with no or fewer obstacles, a visually impaired person usually moves based on travel experience.

However, for the visually impaired people, usually it is difficult to walk in a straight line due to a poor sense of direction. Especially in a scenario like a crosswalk, a road condition is complex, and various motor vehicles and non-motor vehicles cross the road. The visually impaired people face high safety risks in scenarios such as passing a crosswalk based on their travel experience.

### SUMMARY

Embodiments of this application provide an image processing method and a related device. When a user deviates from a crosswalk in a movement process, first indication information is output to improve safety of the user when the user passes through the crosswalk.

A first aspect of embodiments of this application provides an image processing method, where the method is applied to an electronic device, the electronic device is configured to obtain a plurality of frames of images of a front angle of view of a user, and the method includes: determining a first route if a crosswalk is detected in the plurality of frames of images, where the first route is a safe walking route on the crosswalk; obtaining, based on the plurality of frames of images, a second route that the user actually walks; and outputting first indication information to the user if an included angle between the first route and the second route in a same reference system is greater than or equal to a preset angle, where the first indication information indicates that the second route is abnormal. Optionally, a condition for outputting the first indication information may alternatively be that the included angle between the first route and the second route in the same reference system is greater than or equal to the preset angle within a period of time. That is, a case in which inaccurate determining is caused by a measurement error of a frame or shaking of the device is avoided, and frequent output of the first indication information is avoided. In addition, the foregoing method may be used in a scenario in which a user passes through a crosswalk, for example, a scenario in which a blind person passes through a street, and a scenario in which a deaf person passes through a street. The reference system mentioned above may be a virtual reference system, a plane (for example, a ground) reference system, a camera reference system of the electronic device, or the like. This is not specifically limited herein. For example, if the plane is a ground on which the crosswalk is located, the included angle between the first route and the second route in the same reference system may be understood as an included angle between a projection of the first route on the ground and the second route. In addition, the first route may be understood as a safe movement route of the user/the electronic device on the ground, and the second route may be understood as an actual movement route of the user/the electronic device on the ground.

In this embodiment of this application, the included angle between the safe walking route and the actual walking route of the user is determined, and when the included angle is greater than or equal to the preset angle, the first indication information is output to the user, to indicate that an actual walking direction of the user is abnormal. In this way, in a case in which the user deviates from the crosswalk in a movement process, safety of the user is improved by using the output first indication information when the user passes through the crosswalk.

Optionally, in a possible implementation of the first aspect, a first pose in which the electronic device obtains the plurality of frames of images includes a first pitch angle and a first yaw angle, a value range of the first pitch angle is 60 degrees to 100 degrees, and a value range of the first yaw angle is 60 degrees to 120 degrees.

In this possible implementation, the value range of the first pitch angle in the first pose is 60 degrees to 100 degrees, and the value range of the first yaw angle is 60 degrees to 120 degrees. In this way, the pose in which the electronic device captures the image meets a condition, or it is understood that the image captured by the electronic device has reference value for determining the first route and/or the second route only in the foregoing condition. In other words, before the plurality of frames of images are obtained, whether a pose of the electronic device meets the condition may be first determined, and the plurality of frames of images are obtained only when the pose of the electronic device meets the condition, so as to ensure that the obtained plurality of frames of images are reasonable, and the first route and the second route are more accurately determined.

Optionally, in a possible implementation of the first aspect, the steps further include: obtaining a second pose of the electronic device, where a second pitch angle in the second pose is different from the first pitch angle, or a second yaw angle in the second pose is different from the first yaw angle; and outputting second indication information, where the second indication information is used by the user to adjust the second pose to the first pose.

In this possible implementation, when the pose of the electronic device does not meet a photographing condition, the user may adjust the pose of the electronic device by using the second indication information, to adjust the second pose of the electronic device to the first pose or a preset pose range that meets the photographing condition, so that a subsequently obtained image is more reasonable, and the first route and the second route are more accurately determined.

Optionally, in a possible implementation of the first aspect, the first route is an angular bisector in which an included angle formed by extension directions of two edges of the crosswalk is located, or the first route is parallel to the edge of the crosswalk. Further, the first route is parallel to an edge of a short side of a horizontal line (or referred to as a zebra line) in the crosswalk, or extension lines of edges of two short sides of the zebra line intersect to form an angle. A direction that is in an angular bisector and that points to an intersection point is the first route, or the first route is a direction perpendicular to the zebra line. This may be set based on an actual requirement, and is not specifically limited herein.

In this possible implementation, a plurality of manners of determining the first route are provided. When the image includes all or a part of the crosswalk, the first route may be determined.

Optionally, in a possible implementation of the first aspect, before the step of outputting first indication information to the user, the method further includes: determining that an area of the crosswalk in the plurality of frames of images gradually decreases.

In this possible implementation, before sending the first indication information, the electronic device determines that the area of the crosswalk in the plurality of frames of images gradually decreases, to ensure that when the user stands at an intersection of the crosswalk, it is determined whether the user is really going to pass through the crosswalk or walk on a zebra line.

Optionally, in a possible implementation of the first aspect, a movement route in which the electronic device captures the plurality of frames of images is the second route, and the movement route is parallel to the ground on which the crosswalk is located.

In this possible implementation, when the electronic device captures the plurality of frames of images, a route parallel to the ground is the second route, or movement of the electronic device in a dimension parallel to the ground is the second route. A manner of determining the second route is provided.

Optionally, in a possible implementation of the first aspect, after the step of outputting first indication information to the user, the method further includes: outputting third indication information to the user if a new image obtained by the electronic device does not include the crosswalk, where the third indication information indicates that the user has passed through the crosswalk.

In this possible implementation, in a process in which the user moves normally, whether the user has passed through the crosswalk is determined by using a new image. When the new image does not include the crosswalk, the third indication information is sent to remind the user that the user has passed through the crosswalk, thereby improving user experience.

Optionally, in a possible implementation of the first aspect, the plurality of frames of images further include traffic light information, a length of the crosswalk, and motor vehicle information related to the crosswalk; the traffic light information includes a color and duration of a traffic light, and the motor vehicle information includes a traveling direction and a distance of a motor vehicle relative to the crosswalk; and the first indication information further indicates a movement path of the user on the crosswalk.

In this possible implementation, the plurality of frames of images may further include the traffic light information, the length of the crosswalk, the motor vehicle information related to the crosswalk, and the like, so that user safety is ensured by outputting the first indication information.

Optionally, in a possible implementation of the first aspect, before the step of determining a first route, the method further includes: starting a first application based on an operation of the user, where the first application is used by the electronic device to obtain the plurality of frames of images of the front angle of view of the user.

In this possible implementation, obtaining of the plurality of frames of images of the front angle of view of the user may be started by using the operation of the user, to reduce energy consumption caused by a scenario in which the user does not pass the crosswalk.

Optionally, in a possible implementation of the first aspect, a representation form of the first indication information includes a voice and/or mark information.

In this possible implementation, when the user is a blind person, the first indication information may be the voice, that is, the blind person may safely pass through the crosswalk by using the voice of the first indication information. When the user is a deaf user or a user who looks down to view the electronic device, the first indication information may be the mark information, that is, the deaf user or the user who looks down to view the electronic device may safely pass through the crosswalk by using the mark information of the first indication information.

A second aspect of embodiments of this application provides an electronic device, where the electronic device is configured to obtain a plurality of frames of images of a front angle of view of a user, and the electronic device includes: a determining unit, configured to determine a first route if a crosswalk is detected in the plurality of frames of images, where the first route is a safe walking route on the crosswalk; an obtaining unit, configured to obtain, based on the plurality of frames of images, a second route that the user actually walks; and an output unit, configured to output first indication information to the user if an included angle between the first route and the second route in a same reference system is greater than or equal to a preset angle, where the first indication information indicates that the second route is abnormal.

Optionally, in a possible implementation of the second aspect, a first pose in which the electronic device obtains the plurality of frames of images includes a first pitch angle and a first yaw angle, a value range of the first pitch angle is 60 degrees to 100 degrees, and a value range of the first yaw angle is 60 degrees to 120 degrees.

Optionally, in a possible implementation of the second aspect, the obtaining unit is further configured to obtain a second pose of the electronic device, where a second pitch angle in the second pose is different from the first pitch angle, or a second yaw angle in the second pose is different from the first yaw angle; and the output unit is further configured to output second indication information, where the second indication information is used by the user to adjust the second pose to the first pose.

Optionally, in a possible implementation of the second aspect, the first route is an angular bisector in which an included angle formed by extension directions of two edges of the crosswalk is located, or the first route is parallel to the edge of the crosswalk.

Optionally, in a possible implementation of the second aspect, the determining unit is further configured to determine that an area of the crosswalk in the plurality of frames of images gradually decreases.

Optionally, in a possible implementation of the second aspect, a movement route in which the electronic device captures the plurality of frames of images is the second route, and the movement route is parallel to a ground on which the crosswalk is located.

Optionally, in a possible implementation of the second aspect, the output unit is further configured to output third indication information to the user if a new image obtained by the electronic device does not include the crosswalk, where the third indication information indicates that the user has passed through the crosswalk.

Optionally, in a possible implementation of the second aspect, the plurality of frames of images further include traffic light information, a length of the crosswalk, and motor vehicle information related to the crosswalk; the traffic light information includes a color and duration of a traffic light, and the motor vehicle information includes a traveling direction and a distance of a motor vehicle relative to the crosswalk; and the first indication information further indicates a movement path of the user on the crosswalk.

Optionally, in a possible implementation of the second aspect, the electronic device further includes: a starting unit, configured to start a first application based on an operation of the user, where the first application is used by the electronic device to obtain the plurality of frames of images of the front angle of view of the user.

Optionally, in a possible implementation of the second aspect, a representation form of the first indication information includes a voice and/or mark information.

A third aspect of embodiments of this application provides an electronic device, including a processor. The processor is coupled to a memory, the memory is configured to store a program or instructions, and when the program or instructions is/are executed by the processor, the electronic device is enabled to implement the method according to the first aspect or any possible implementation of the first aspect.

A fourth aspect of embodiments of this application provides a computer-readable medium. The computer-readable medium stores a computer program or instructions. When the computer program or the instructions is/are run on a computer, the computer is enabled to perform the method in the first aspect or any possible implementation of the first aspect.

A fifth aspect of embodiments of this application provides a computer program product. When the computer program product is executed on a computer, the computer is enabled to perform the method according to the first aspect or any possible implementation of the first aspect.

For technical effects brought by the second aspect, the third aspect, the fourth aspect, the fifth aspect, or any possible implementation thereof, refer to technical effects brought by the first aspect or different possible implementations of the first aspect. Details are not described herein again.

It can be learned from the foregoing technical solutions that embodiments of this application have the following advantages: In embodiments of this application, an included angle between a safe walking route and an actual walking route of a user is determined, and when the included angle is greater than or equal to a preset angle, first indication information is output to the user, to indicate that an actual walking direction of the user is abnormal. In this way, when the user deviates from a crosswalk in a moving process, safety of the user is improved by using the output first indication information.

### BRIEF DESCRIPTION OF DRAWINGS

FIG. 1 is a schematic diagram of an application scenario according to an embodiment of the present invention;
FIG. 2 is a schematic diagram of a structure of an electronic device according to an embodiment of this application;
FIG. 3 is a schematic flowchart of an image processing method according to an embodiment of this application;
FIG. 4 is a schematic diagram of an example of an image according to an embodiment of this application;
FIG. 5 is a schematic diagram of an example of another image according to an embodiment of this application;
FIG. 6 is a schematic diagram of an example of a crosswalk according to an embodiment of this application;
FIG. 7 is a schematic diagram of an example of another crosswalk according to an embodiment of this application;
FIG. 8 is a schematic diagram of an example of another crosswalk according to an embodiment of this application;
FIG. 9 is a schematic diagram of an example of a posture angle of an electronic device according to an embodiment of this application;
FIG. 10 is a schematic diagram of an example of a first route and a second route according to an embodiment of this application;
FIG. 11 is a schematic diagram of an example of another first route and another second route according to an embodiment of this application;
FIG. 12 is a schematic diagram of an example of first indication information according to an embodiment of this application;
FIG. 13 is a schematic diagram of an example of a scenario in which an electronic device is in a second pose according to an embodiment of this application;
FIG. 14 is a schematic diagram of an example of a scenario in which an electronic device is in a first pose according to an embodiment of this application;
FIG. 15 is a schematic diagram of an example of a smart recognition function in an electronic device according to an embodiment of this application.
FIG. 16 is a schematic diagram of another structure of an electronic device according to an embodiment of this application; and
FIG. 17 is a schematic diagram of another structure of an electronic device according to an embodiment of this application.

### DESCRIPTION OF EMBODIMENTS

Embodiments of this application provide an image processing method and a related device. When a user deviates from a crosswalk in a movement process, first indication information is output to improve safety of the user when the user passes through the crosswalk.

Currently, visually impaired people mainly rely on a blind cane to recognize an obstacle on a road. Because the blind cane is mainly used for obstacles in front of the road, in a scenario like a crosswalk with no or fewer obstacles, a visually impaired person usually moves based on travel experience. However, for the visually impaired people, usually it is difficult to walk in a straight line due to a poor sense of direction. Especially in a scenario like a crosswalk, a road condition is complex, and various motor vehicles and non-motor vehicles cross the road. The visually impaired people face high safety risks in scenarios such as passing a crosswalk based on their travel experience.

To resolve the foregoing technical problem, embodiments of this application provide an interface display method and an electronic device. An included angle between a safe walking route and an actual walking route of a user is determined, and when the included angle is greater than or equal to a preset angle, first indication information is output to the user, to indicate that an actual walking direction of the user is abnormal. In this way, in a case in which the user deviates from a crosswalk in a movement process, safety of the user is improved by using the output first indication information when the user passes through the crosswalk.

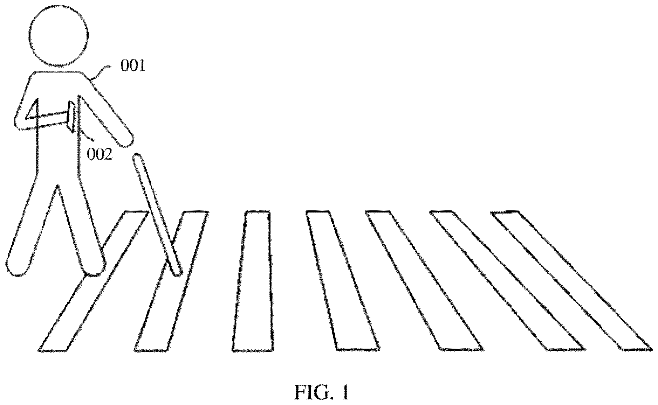

Before the method provided in embodiments of this application is described, an application scenario of the method provided in embodiments of this application is first described. An application scenario of the method provided in embodiments of this application may be as shown in FIG. 1. The application scenario includes a user 001 and an electronic device 002.

The user 001 uses the electronic device 002 to assist in passing through a crosswalk (or referred to as a road, a zebra crossing, or the like). The user 001 may be a visually impaired user, or may be a normal user. In other words, the user may be a blind user, a deaf user, or a normal user.

The electronic device 002 is equipped with an operating system and an application (for example, intelligent recognition). By using an operation of the user 001, an image may be captured, a crosswalk (for example, a zebra crossing) in the image may be recognized, indication information (for example, warning information and correction information) may be sent, and the like. The indication information may be audio information, a visual image, or the like. This is not specifically limited herein.

With reference to the accompanying drawings, the following describes in detail an interface display method and an electronic device according to embodiments of this application.

First, the electronic device is described. The electronic device in embodiments of this application may be a digital display product, for example, a mobile phone, a smartwatch, smart glasses, a smart band, a tablet computer (pad), a portable game console, a palmtop computer (personal digital assistant, PDA), a notebook computer, an ultra mobile personal computer (ultra mobile personal computer, UMPC), a handheld computer, a netbook, an in-vehicle media playing device, a wearable electronic device (for example, a watch, a band, or glasses), a virtual reality (virtual reality, VR) terminal device, or an augmented reality (augmented reality, AR) terminal device. An example in which the electronic device is a mobile phone is used for description in this embodiment of this application.

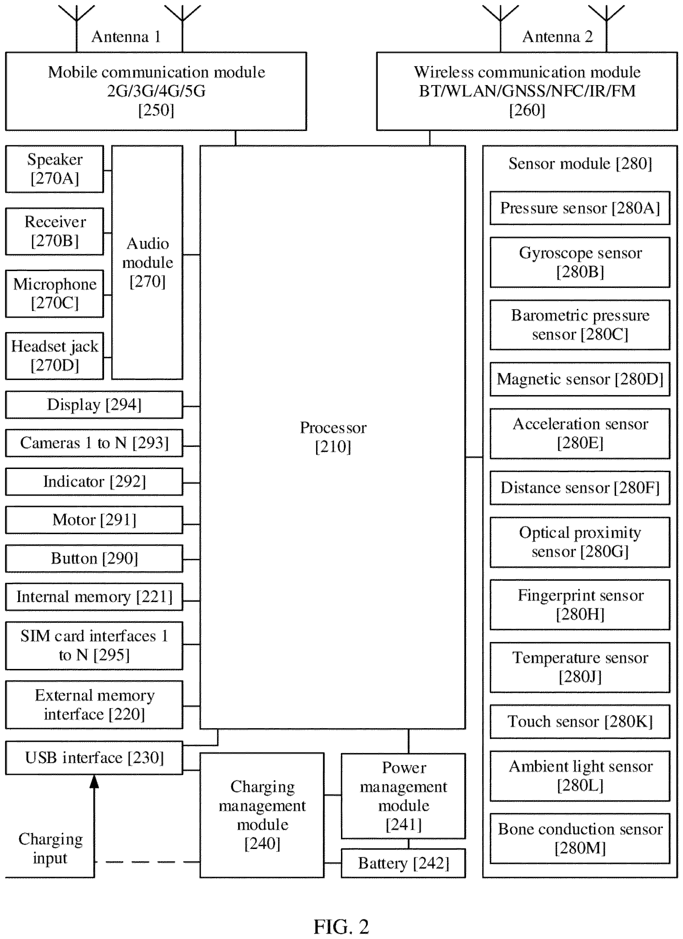

FIG. 2 is a schematic diagram of a structure of an electronic device according to an embodiment of this application. As shown in FIG. 2, the electronic device may include a processor 210, an external memory interface 220, an internal memory 221, a universal serial bus (universal serial bus, USB) interface 230, a charging management module 240, a power management module 241, a battery 242, an antenna 1, an antenna 2, a mobile communication module 250, a wireless communication module 260, an audio module 270, a speaker 270A, a receiver 270B, a microphone 270C, a headset jack 270D, a sensor module 280, a button 290, a motor 291, an indicator 292, a camera 293, a display 294, a subscriber identification module (subscriber identification module, SIM) card interface 295, and the like. The sensor module 280 may include a pressure sensor 280A, a gyroscope sensor 280B, a barometric pressure sensor 280C, a magnetic sensor 280D, an acceleration sensor 280E, a distance sensor 280F, an optical proximity sensor 280G, a fingerprint sensor 280H, a temperature sensor 280J, a touch sensor 280K, an ambient light sensor 280L, a bone conduction sensor 280M, and the like.

It may be understood that the structure shown in this embodiment does not constitute a specific limitation on the electronic device. In some other embodiments, the electronic device may include more or fewer components than those shown in the figure, or may combine some components, or may split some components, or may have different component arrangements. The components shown in the figure may be implemented by hardware, software, or a combination of software and hardware.

The processor 210 may include one or more processing units. For example, the processor 210 may include an application processor (application processor, AP), a modem processor, a graphics processing unit (graphics processing unit, GPU), an image signal processor (image signal processor, ISP), a controller, a memory, a video codec, a digital signal processor (digital signal processor, DSP), a baseband processor, and/or a neural-network processing unit (neural-network processing unit, NPU). Different processing units may be independent components, or may be integrated into one or more processors.

The controller may be a nerve center and a command center of the electronic device. The controller may generate an operation control signal based on an instruction operation code and a time sequence signal, to complete control of instruction reading and instruction execution.

A memory may be further disposed in the processor 210, and is configured to store instructions and data. In some embodiments, the memory in the processor 210 is a cache. The memory may store instructions or data just used or cyclically used by the processor 210. If the processor 210 needs to use the instructions or the data again, the processor 210 may directly invoke the instructions or the data from the memory. This avoids repeated access and reduces a waiting time of the processor 210, thereby improving system efficiency.

In some embodiments, the processor 210 may include one or more interfaces. The interface may include an inter-integrated circuit (inter-integrated circuit, I2C) interface, an inter-integrated circuit sound (inter-integrated circuit sound, I2S) interface, a pulse code modulation (pulse code modulation, PCM) interface, a universal asynchronous receiver/transmitter (universal asynchronous receiver/transmitter, UART) interface, a mobile industry processor interface (mobile industry processor interface, MIPI), a general-purpose input/output (general-purpose input/output, GPIO) interface, a subscriber identity module (subscriber identity module, SIM) interface, a universal serial bus (universal serial bus, USB) interface, and/or the like.

It may be understood that an interface connection relationship between the modules shown in this embodiment is merely used as an example for description, and does not constitute a limitation on the structure of the electronic device. In some other embodiments, the electronic device may alternatively use an interface connection manner different from that in the foregoing embodiment, or use a combination of a plurality of interface connection manners.

The charging management module 240 is configured to receive a charging input from a charger. The charger may be a wireless charger or a wired charger. In some embodiments of wired charging, the charging management module 240 may receive a charging input of a wired charger through the USB interface 230. In some embodiments of wireless charging, the charging management module 240 may receive a wireless charging input through a wireless charging coil of the electronic device. When charging the battery 242, the charging management module 240 may further supply power to the electronic device by using the power management module 241.

The power management module 241 is configured to connect to the battery 242, the charging management module 240, and the processor 210. The power management module 241 receives an input from the battery 242 and/or an input from the charging management module 240, and supplies power to the processor 210, the internal memory 221, an external memory, the display 294, the camera 293, the wireless communication module 260, and the like. The power management module 241 may be further configured to monitor parameters such as a battery capacity, a battery cycle count, and a battery health status (an electric leakage or impedance). In some other embodiments, the power management module 241 may alternatively be disposed in the processor 210. In some other embodiments, the power management module 241 and the charging management module 240 may alternatively be disposed in a same device.

A wireless communication function of the electronic device may be implemented by using the antenna 1, the antenna 2, the mobile communication module 250, the wireless communication module 260, the modem processor, the baseband processor, and the like.

The antenna 1 and the antenna 2 are configured to transmit and receive an electromagnetic wave signal. Each antenna of the electronic device may be configured to cover one or more communication frequency bands. Different antennas may be further multiplexed, to improve antenna utilization. For example, the antenna 1 may be multiplexed as a diversity antenna of a wireless local area network. In some other embodiments, the antenna may be used in combination with a tuning switch.

The mobile communication module 250 may provide a solution that is applied to the electronic device and that includes wireless communication such as 2G/3G/4G/5G. The mobile communication module 250 may include at least one filter, a switch, a power amplifier, a low noise amplifier (low noise amplifier, LNA), and the like. The mobile communication module 250 may receive an electromagnetic wave through the antenna 1, perform processing such as filtering or amplification on the received electromagnetic wave, and transmit a processed electromagnetic wave to the modem processor for demodulation. The mobile communication module 250 may further amplify a signal modulated by the modem processor, and convert an amplified signal into an electromagnetic wave for radiation through the antenna 1. In some embodiments, at least some function modules in the mobile communication module 250 may be disposed in the processor 210. In some embodiments, at least some function modules in the mobile communication module 250 may be disposed in a same device as at least some modules of the processor 210.

The modem processor may include a modulator and a demodulator. The modulator is configured to modulate a to-be-sent low-frequency baseband signal into a medium-high frequency signal. The demodulator is configured to demodulate a received electromagnetic wave signal into a low-frequency baseband signal. Then, the demodulator transfers the low-frequency baseband signal obtained through demodulation to the baseband processor for processing. After being processed by the baseband processor, the low-frequency baseband signal is transmitted to the application processor. The application processor outputs a sound signal by using an audio device (which is not limited to the speaker 270A, the receiver 270B, and the like), or displays an image or a video through the display 294. In some embodiments, the modem processor may be an independent device. In some other embodiments, the modem processor may be independent of the processor 210, and is disposed in a same device as the mobile communication module 250 or another function module.

The wireless communication module 260 may provide a solution applied to the electronic device for wireless communication including a wireless local area network (wireless local area network, WLAN) (for example, a wireless fidelity (wireless fidelity, Wi-Fi) network), Bluetooth (Bluetooth, BT), a global navigation satellite system (global navigation satellite system, GNSS), frequency modulation (frequency modulation, FM), near field communication (near field communication, NFC), an infrared (infrared, IR) technology, and the like. The wireless communication module 260 may be one or more devices integrating at least one communication processing module. The wireless communication module 260 receives an electromagnetic wave through the antenna 2, performs frequency modulation and filtering processing on the electromagnetic wave signal, and sends a processed signal to the processor 210. The wireless communication module 260 may further receive a to-be-sent signal from the processor 210, perform frequency modulation and amplification on the signal, and convert the signal into an electromagnetic wave for radiation through the antenna 2.

In some embodiments, in the electronic device, the antenna 1 is coupled to the mobile communication module 250, and the antenna 2 is coupled to the wireless communication module 260, so that the electronic device can communicate with a network and another device by using a wireless communication technology. The wireless communication technology may include a global system for mobile communications (global system for mobile communications, GSM), a general packet radio service (general packet radio service, GPRS), code division multiple access (code division multiple access, CDMA), wideband code division multiple access (wideband code division multiple access, WCDMA), time-division code division multiple access (time-division code division multiple access, TDSCDMA), long term evolution (long term evolution, LTE), BT, a GNSS, a WLAN, NFC, FM, an IR technology, and/or the like. The GNSS may include a global positioning system (global positioning system, GPS), a global navigation satellite system (global navigation satellite system, GLONASS), a BeiDou navigation satellite system (BeiDou navigation satellite system, BDS), a quasi-zenith satellite system (quasi-zenith satellite system, QZSS), and/or a satellite based augmentation system (satellite based augmentation system, SBAS).

The electronic device implements a display function by using the GPU, the display 294, the application processor, and the like. The GPU is a microprocessor for image processing, and is connected to the display 294 and the application processor. The GPU is configured to: perform mathematical and geometric computation, and render an image. The processor 210 may include one or more GPUs that execute program instructions to generate or change displayed information.

The display 294 is configured to display an image, a video, or the like. The display 294 includes a display panel. The display panel may use a liquid crystal display (liquid crystal display, LCD), an organic light-emitting diode (organic light-emitting diode, OLED), an active-matrix organic light emitting diode (active-matrix organic light emitting diode, AMOLED), a flex light-emitting diode (flex light-emitting diode, FLED), a Miniled, a MicroLed, a Micro-oLed, a quantum dot light emitting diode (quantum dot light emitting diode, QLED), and the like.

The electronic device may implement a photographing function by using the ISP, the camera 293, the video codec, the GPU, the display 294, the application processor, and the like.

The ISP is configured to process data fed back by the camera 293. For example, during photographing, a shutter is pressed, and light is transmitted to a photosensitive element of the camera through a lens. An optical signal is converted into an electrical signal, and the photosensitive element of the camera transmits the electrical signal to the ISP for processing, to convert the electrical signal into a visible image. The ISP may further perform algorithm optimization on noise, brightness, and complexion of the image. The ISP may further optimize parameters such as exposure and a color temperature of a photographing scenario. In some embodiments, the ISP may be disposed in the camera 293.

The camera 293 is configured to capture a static image or a video. An optical image of an object is generated through the lens, and is projected to the photosensitive element. The photosensitive element may be a charge coupled device (charge coupled device, CCD) or a complementary metal-oxide-semiconductor (complementary metal-oxide-semiconductor, CMOS) optoelectronic transistor. The light-sensitive element converts an optical signal into an electrical signal, and then transmits the electrical signal to the ISP to convert the electrical signal into a digital image signal. The ISP outputs the digital image signal to the DSP for processing. The DSP converts the digital image signal into a standard image signal in a format like RGB or YUV. In some embodiments, the electronic device may include one or N cameras 293, where N is a positive integer greater than 1.

The camera 293 may be further configured to perceive external environment and a user action, so that the electronic device provides personalized and scenario-based service experience for a user. The camera 293 can obtain rich and accurate information, so that the electronic device senses the external environment and the user action. Specifically, in this embodiment of this application, the camera 293 may be configured to identify whether a user of the electronic device is a first user or a second user.

The digital signal processor is configured to process a digital signal, and may process another digital signal in addition to a digital image signal. For example, when the electronic device selects a frequency, the digital signal processor is configured to perform Fourier transform and the like on frequency energy.

The video codec is configured to compress or decompress a digital video. The electronic device may support one or more types of video codecs. Therefore, the electronic device may play or record videos in a plurality of coding formats, for example, moving picture experts group (moving picture experts group, MPEG)-1, MPEG-2, MPEG-3, and MPEG-4.

The NPU is a neural-network (neural-network, NN) computing processor, and simulates a biological neural network structure like a transmission mode between neurons in a human brain to perform rapid processing on input information, and can perform continuous self-learning. Applications such as intelligent cognition of the electronic device, for example, image recognition, facial recognition, speech recognition, and text understanding, may be implemented through the NPU.

The external memory interface 220 may be configured to connect to an external storage card, for example, a micro SD card, to expand a storage capability of the electronic device. The external storage card communicates with the processor 210 through the external memory interface 220, to implement a data storage function. For example, a music file or a video file is stored in the external storage card.

The internal memory 221 may be configured to store computer-executable program code, and the executable program code includes instructions. The processor 210 executes various function applications of the electronic device and data processing by running the instructions stored in the internal memory 221. For example, in this embodiment of this application, the processor 210 may execute the instructions stored in the internal memory 221, to display corresponding display content on the display in response to an operation performed by the user on the display 294. The internal memory 221 may include a program storage area and a data storage area. The program storage area may store an operating system, an application required by at least one function (for example, a sound playing function or an image playing function), and the like. The data storage area may store data (for example, audio data and a phone book) created when the electronic device is used, and the like. In addition, the internal memory 221 may include a highspeed random access memory, and may further include a non-volatile memory, for example, at least one magnetic disk storage device, a flash memory device, or a universal flash memory (universal flash storage, UFS).

The electronic device may implement an audio function by using the audio module 270, the speaker 270A, the receiver 270B, the microphone 270C, the headset jack 270D, the application processor, and the like, for example, music playing and recording.

The audio module 270 is configured to convert digital audio information into an analog audio signal for output, and is further configured to convert an analog audio input into a digital audio signal. The audio module 270 may be further configured to: code and decode an audio signal. In some embodiments, the audio module 270 may be disposed in the processor 210, or some function modules in the audio module 270 may be disposed in the processor 210. The speaker 270A, also referred to as a "horn", is configured to convert an audio electrical signal into a sound signal. The electronic device may listen to music or listen to a hands-free call through the speaker 270A. The receiver 270B, also referred to as an "earpiece", is configured to convert an audio electrical signal into a sound signal. When the electronic device answers a call or voice information, the receiver 270B may be placed close to a human ear to answer the voice. The microphone 270C, also referred to as a "mike" or a "mic", is configured to convert a sound signal into an electrical signal. When the user makes a call or sends voice information, or when the electronic device needs to be triggered to perform some functions by using a voice assistant, the user may make a sound by using a human mouth close to the microphone 270C, and enter a sound signal to the microphone 270C. At least one microphone 270C may be disposed in the electronic device. In some other embodiments, two microphones 270C may be disposed in the electronic device, to capture a sound signal and implement a noise reduction function. In some other embodiments, three, four, or more microphones 270C may alternatively be disposed in the electronic device, to capture a sound signal, implement noise reduction, identify a sound source, implement a directional recording function, and the like.

The headset jack 270D is configured to connect to a wired headset. The headset jack 270D may be the USB interface 230, or may be a 3.5 mm open mobile terminal platform (open mobile terminal platform, OMTP) standard interface or a cellular telecommunications industry association of the USA (cellular telecommunications industry association of the USA, CTIA) standard interface.

The pressure sensor 280A is configured to sense a pressure signal, and can convert the pressure signal into an electrical signal. In some embodiments, the pressure sensor 280A may be disposed on the display 294. There are many types of pressure sensors 280A, for example, a resistive pressure sensor, an inductive pressure sensor, and a capacitive pressure sensor. The capacitive pressure sensor may include at least two parallel plates made of conductive materials. When a force is applied to the pressure sensor 280A, a capacitance between electrodes changes. The electronic device determines a pressure strength based on the change of the capacitance. When a touch operation is performed on the display 294, the electronic device detects a strength of the touch operation based on the pressure sensor 280A. The electronic device may further calculate a touch location based on a detection signal of the pressure sensor 280A. In some embodiments, touch operations that are performed in a same touch location but have different touch operation strengths may correspond to different operation instructions. For example, when a touch operation whose touch operation strength is less than a pressure threshold is performed on an SMS message application icon, an instruction for viewing an SMS message is executed. When a touch operation whose touch operation strength is greater than or equal to the pressure threshold is performed on the SMS message application icon, an instruction for creating a new SMS message is executed.

The gyroscope sensor 280B may be configured to determine a motion posture of the electronic device. In some embodiments, an angular velocity of the electronic device around three axes (namely, axes x, y, and z) may be determined through the gyroscope sensor 280B. The gyroscope sensor 280B may be configured for photographing image stabilization. For example, when the shutter is pressed, the gyroscope sensor 280B detects an angle at which the electronic device jitters, calculates, based on the angle, a distance for which a lens module needs to compensate, and allows the lens to cancel the jitter of the electronic device through reverse motion, to implement image stabilization. The gyroscope sensor 280B may be further configured for navigation and motion-controlled gaming scenarios. In addition, the gyroscope sensor 280B may be further configured to measure a rotation amplitude or a moving distance of the electronic device.

The barometric pressure sensor 280C is configured to measure atmospheric pressure. In some embodiments, the electronic device calculates an altitude by using atmospheric pressure measured by the barometric pressure sensor 280C, to assist in positioning and navigation.

The magnetic sensor 280D includes a Hall sensor. The electronic device may detect opening and closing of a flip cover leather case by using the magnetic sensor 280D. In some embodiments, when the electronic device is a flip phone, the electronic device may detect opening and closing of a flip cover based on the magnetic sensor 280D. Further, a feature like automatic unlocking of the flip cover is set based on a detected opening or closing state of the leather case or a detected opening or closing state of the flip cover.

The acceleration sensor 280E may detect magnitudes of accelerations of the electronic device in all directions (usually on three axes). When the electronic device is static, magnitude and a direction of gravity may be detected. The acceleration sensor 280E may be further configured to identify a posture of the electronic device, and is used in an application such as switching between a landscape mode and a portrait mode or a pedometer. In addition, the acceleration sensor 280E may be further configured to measure an orientation (namely, a direction vector of the orientation) of the electronic device.

The distance sensor 280F is configured to measure a distance. The electronic device may measure a distance through infrared or laser. In some embodiments, in an image shooting scenario, the electronic device may measure a distance by using the distance sensor 280F, to implement fast focusing.

The optical proximity sensor 280G may include, for example, a light emitting diode (LED) and an optical detector, for example, a photodiode. The light emitting diode may be an infrared light emitting diode. The electronic device emits infrared light outwards through the light emitting diode. The electronic device uses the photodiode to detect infrared reflected light from a nearby object. When detecting sufficient reflected light, the electronic device may determine that there is an object near the electronic device. When insufficient reflected light is detected, the electronic device may determine that there is no object near the electronic device. The electronic device may detect, by using the optical proximity sensor 280G, that the user holds the electronic device close to an ear for a call, to automatically turn off the screen to save power. The optical proximity sensor 280G may further be configured in a leather case mode or a pocket mode to automatically unlock and lock the screen.

The ambient light sensor 280L is configured to sense ambient light brightness. The electronic device may adaptively adjust brightness of the display 294 based on the sensed ambient light brightness. The ambient light sensor 280L may further be configured to automatically adjust white balance during photographing. The ambient light sensor 280L may further cooperate with the optical proximity sensor 280G to detect whether the electronic device is in a pocket, to avoid an unintentional touch.

The fingerprint sensor 280H is configured to collect a fingerprint. The electronic device may implement fingerprint unlocking, application lock access, fingerprint photographing, incoming call answering, and the like by using a collected fingerprint feature.

The temperature sensor 280J is configured to detect a temperature. In some embodiments, the electronic device executes a temperature processing policy by using the temperature detected by the temperature sensor 280J. For example, when a temperature reported by the temperature sensor 280J exceeds a threshold, the electronic device reduces performance of a processor located near the temperature sensor 280J, to reduce power consumption and implement thermal protection. In some other embodiments, when the temperature is lower than another threshold, the electronic device heats the battery 242 to avoid abnormal shutdown of the electronic device that is caused due to a low temperature. In some other embodiments, when the temperature is lower than still another threshold, the electronic device boosts an output voltage of the battery 242, to avoid abnormal shutdown caused by a low temperature.

The touch sensor 280K is also referred to as a "touch panel". The touch sensor 280K may be disposed on the display 294. The touch sensor 280K and the display 294 form a touchscreen. The touch sensor 280K is configured to detect a touch operation performed on or near the touch sensor. The touch sensor may transfer the detected touch operation to the application processor to determine a type of the touch event. The display 294 may provide a visual output related to the touch operation. In some other embodiments, the touch sensor 280K may alternatively be disposed on a surface of the electronic device, and a location of the touch sensor 280K is different from that of the display 294.

The bone conduction sensor 280M may obtain a vibration signal. In some embodiments, the bone conduction sensor 280M may obtain a vibration signal of a vibration bone block of a human vocal part. The bone conduction sensor 280M may further contact a human pulse, and receive a blood pressure beat signal. In some embodiments, the bone conduction sensor 280M may alternatively be disposed in a headset, to constitute a bone conduction headset. The audio module 270 may obtain a voice signal through parsing based on the vibration signal that is of the vibration bone of the vocal part and that is obtained by the bone conduction sensor 280M, to implement a voice function. The application processor may parse heart rate information based on a blood pressure beat signal obtained by the bone conduction sensor 280M, to implement a heart rate detection function.

The button 290 includes a power button, a volume button, or the like. The button 290 may be a mechanical button, or may be a touch button. The electronic device may receive a button input, and generate a button signal input related to user setting and function control of the electronic device.

The electronic device uses various sensors, buttons 290, and/or cameras 293 in the sensor module 280.

The motor 291 may generate a vibration prompt. The motor 291 may be configured to provide an incoming call vibration prompt and a touch vibration feedback. For example, touch operations performed on different applications (for example, photographing and audio playing) may correspond to different vibration feedback effects. The motor 291 may further correspond to different vibration feedback effects for touch operations performed on different areas of the display 294. Different application scenarios (for example, a time reminder, information receiving, an alarm clock, and a game) may also correspond to different vibration feedback effects. The touch vibration feedback effect may be further customized.

The indicator 292 may be an indicator light, and may be configured to indicate a charging status and a power change, or may be configured to indicate a message, a missed call, a notification, and the like.

The SIM card interface 295 is configured to connect to a SIM card. The SIM card may be inserted into the SIM card interface 295 or removed from the SIM card interface 295, to implement contact with and separation from the electronic device. The electronic device may support one or N SIM card interfaces, and N is a positive integer greater than 1. The SIM card interface 295 may support a nano-SIM card, a micro-SIM card, a SIM card, and the like. A plurality of cards may be simultaneously inserted into a same SIM card interface 295. The plurality of cards may be of a same type or different types. The SIM card interface 295 is further compatible with different types of SIM cards. The SIM card interface 295 is further compatible with an external storage card. The electronic device interacts with a network through the SIM card, to implement functions such as a call and data communication. In some embodiments, the electronic device uses eSIM, that is, an embedded SIM card. The eSIM card may be embedded into the electronic device, and cannot be separated from the electronic device.

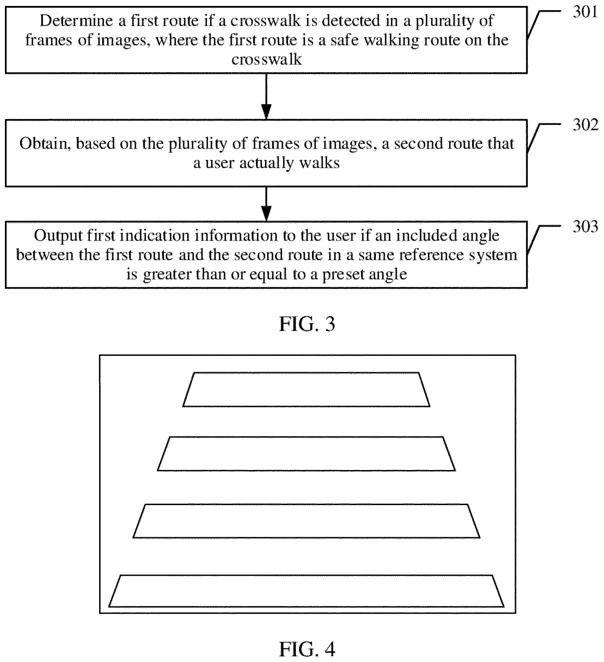

The following describes an image processing method provided in an embodiment of this application by using an example in which the electronic device is a mobile phone. The method may be performed by an electronic device, or may be performed by a component (for example, a processor, a chip, or a chip system) of the electronic device. The electronic device is configured to obtain a plurality of frames of images of a front angle of view of a user. As shown in FIG. 3, the image processing method may include step 301 to step 303, which are separately described below.

Step 301: Determine a first route if a crosswalk is detected in a plurality of frames of images, where the first route is a safe walking route on the crosswalk.

In this embodiment of this application, the electronic device may obtain the plurality of frames of images in a plurality of manners. The plurality of frames of images may be captured/photographed by the electronic device, may be sent by another device (another device connected to the electronic device) and received by the electronic device, may be selected by the electronic device from a database, or the like. This is not specifically limited herein. This specification is described by using only an example in which the electronic device obtains a plurality of frames of images of a front angle of view of the user in real time. In actual application, the electronic device may periodically obtain the plurality of frames of images of the front angle of view of the user. This is not specifically limited herein.

Optionally, if the electronic device is used in a scenario in which a pedestrian passes through a crosswalk, the electronic device may be a portable electronic device carried by the pedestrian, for example, a mobile phone, a smartwatch, or smart glasses.

The first route is determined if the crosswalk is detected in the plurality of frames of images, where the first route is the safe walking route on the crosswalk.

There are a plurality of cases of the first route in this embodiment of this application. The first route may be an extension direction of the crosswalk, or may be understood as a direction perpendicular to a horizontal line in the crosswalk, or the like. This is not specifically limited herein. It should be noted that the first route may be not only one route, but may be a plurality of routes within a specific range, for example, the first route is a plurality of routes that are led out from an intersection point within an "angle" range formed by horizontal lines on two sides of the crosswalk.

When the first route is the extension direction of the crosswalk, determining of the first route is related to an integrity degree of the crosswalk in the image. In a possible implementation, the image includes a complete crosswalk, edge extension lines of the two sides of the crosswalk intersect to form an angle, and a direction that is on an angular bisector of the angle and that points to an intersection point is the first route (that is, the first route is an angular bisector in which an included angle formed by edge extension directions of the two sides of the crosswalk is located). In another possible implementation, the image includes an incomplete crosswalk, and the first route is parallel to an edge of the crosswalk, or a direction that is perpendicular to a crosswalk line and that is in a direction away from a location of the electronic device is the first route. This is not specifically limited herein.

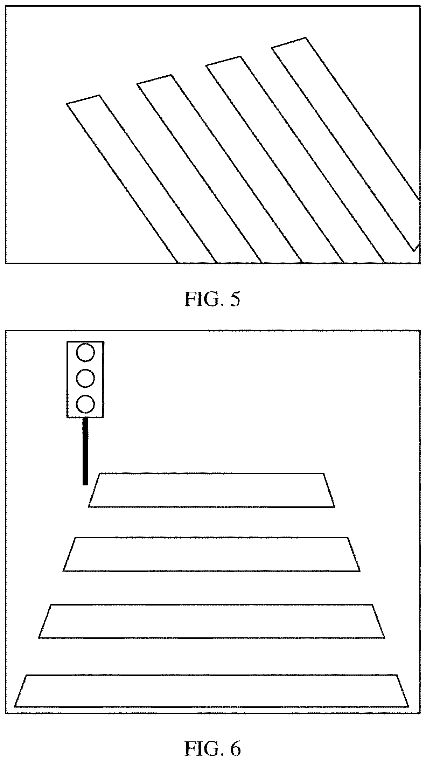

For example, when the image includes a complete crosswalk, the image may be as shown in FIG. 4. When the image includes a part of the crosswalk, the image may be as shown in FIG. 5. It may be understood that the plurality of frames of images obtained by the electronic device may include the images in FIG. 4 and FIG. 5, or may include the image in FIG. 4 or FIG. 5. This is not specifically limited herein.

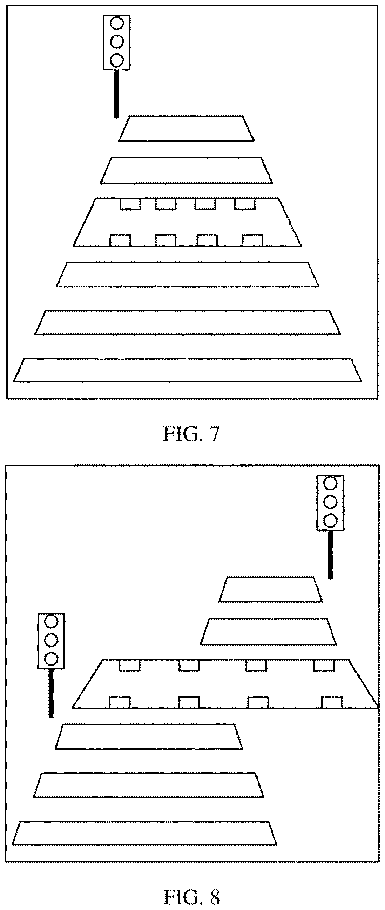

In this embodiment of this application, there are a plurality of cases of the crosswalk. The crosswalk may be located at an intersection without a traffic light (for example, shown in FIG. 4), may be located at an intersection with only one traffic light (for example, shown in FIG. 6), may be located at an intersection with only one traffic light and a safety island in the middle (for example, shown in FIG. 7), or may be located at an intersection with two traffic lights and a safety island in the middle (for example, shown in FIG. 8), or the like. A specific case of the crosswalk may be set based on an actual requirement. This is not specifically limited herein.

In addition, for a length, a width, a line spacing, and the like of the crosswalk in this embodiment of this application, refer to a specification in the specification for setting urban road traffic signs and marking lines (GB 51038-2015).

Optionally, a crosswalk line needs to be a group of white parallel thick solid lines. A line width needs to be 40 cm or 45 cm, and a line spacing needs to be 60 cm. A maximum line spacing should not exceed 80 cm. A width of the crosswalk line should be greater than or equal to 3 m, and needs to be increased by 1 m at each level.

Optionally, the width, a form, and a location of the crosswalk line needs to meet the following requirements: When the crosswalk line is longer than 16 m, a safety island needs to be disposed at a separator or a boundary line of an opposite lane. A length of the safety island should not be less than the width of the crosswalk line. A width of the safety island should not be less than 2 m, and should not be less than 1.5 m when there is a difficulty. The safety island needs to be installed with flexible traffic pillars, safety protection facilities, and the like.

The plurality of frames of images in this embodiment of this application include an object, the object includes the crosswalk, and the crosswalk is used by a pedestrian to cross a vehicle lane. It may be understood that an image in the plurality of frames of images may include a complete crosswalk or a part of the crosswalk. One image in the plurality of frames of images may include a complete crosswalk, and another image may include a part of the crosswalk, and the like. This is not specifically limited herein.

Optionally, the object in the plurality of frames of images may further include traffic light information, the length of the crosswalk, motor vehicle information related to the crosswalk, and the like. The traffic light information may include a color, duration, and the like of a traffic light, and the motor vehicle information includes a traveling direction, a distance, and the like of a motor vehicle relative to the lane.

Optionally, an area of the crosswalk in the plurality of frames of images may gradually decrease. In this case, it may be understood as that the user is deviated from the crosswalk.

Step 302: Obtain, based on the plurality of frames of images, a second route that the user actually walks.

In this embodiment of this application, the second route may be determined in a plurality of cases. The second route may be a movement route of the electronic device parallel to a ground on which the crosswalk is located, or may be a movement route of the user holding the electronic device, or may be a route of a direction towards which the electronic device is located, or may be a route that is on a midline of the image and that is away from the location of the electronic device. This is not specifically limited herein.

Optionally, the second route may be determined based on a pose in which the electronic device captures the image. In this case, the electronic device may detect the pose of the electronic device by using a gyroscope and/or an acceleration sensor.

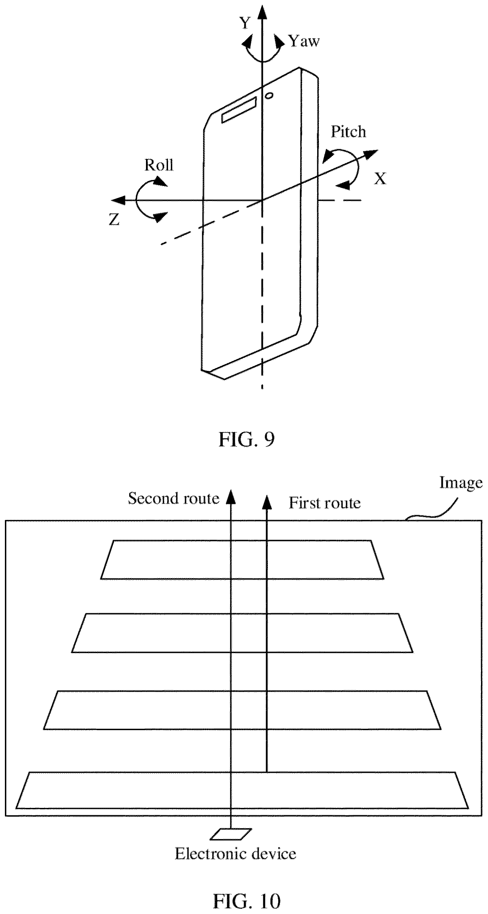

In this embodiment of this application, a pose orientation of the electronic device may be a posture angle of the electronic device. The posture angle may include a roll angle and a yaw angle (or referred to as a tilt angle), or the posture angle includes a roll angle, a yaw angle, and a pitch angle. The roll angle represents an angle around a z-axis, the yaw angle represents an angle around a y-axis, and the pitch angle represents an angle around an x-axis. A relationship between an orientation of the electronic device and the x-axis, the y-axis, and the z-axis may be shown in FIG. 9.

For example, the image shown in FIG. 4 is still used as an example. The first route and the second route may be as shown in FIG. 10. The second route is a route in which the orientation of the electronic device is located, and the first route is a route that is perpendicular to the crosswalk line and that is away from the location of the electronic device. In this way, it may be determined that an included angle between the first route and the second route in a same reference system (for example, a plane on which the crosswalk is located) is 0 degrees.

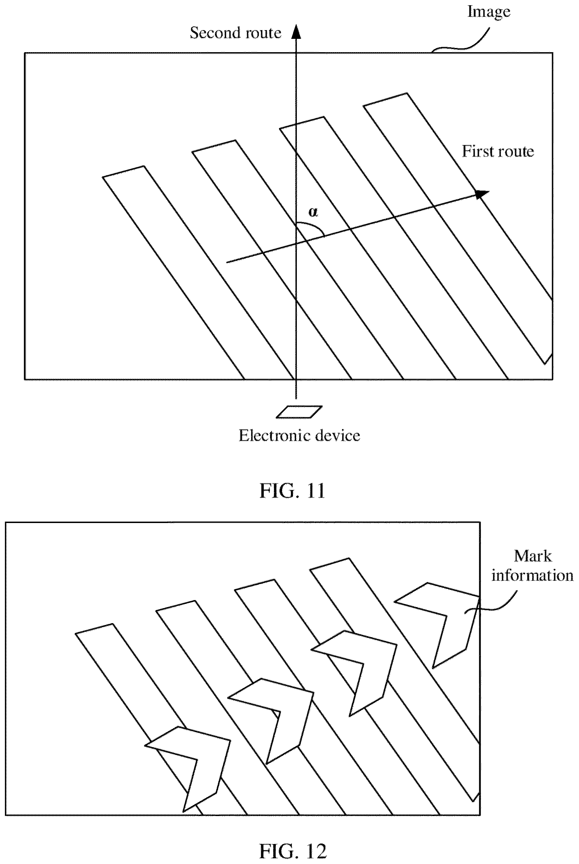

For example, the image shown in FIG. 5 is still used as an example. The first route and the second route may be as shown in FIG. 11. The second route is a route in which the orientation of the electronic device is located, and the first route is a route that is perpendicular to the crosswalk line and that is away from the location of the electronic device. In this way, it may be determined that the included angle between the first route and the second route is a.

The first route and the second route in this embodiment of this application may be presented on the plurality of frames of images, and may be specifically carried on each image of the plurality of frames of images. In addition, the first route and the second route may be presented to the user in a dynamic/static image manner or a voice manner. This is not specifically limited herein.

Step 303: Output first indication information to the user if an included angle between the first route and the second route in a same reference system is greater than or equal to a preset angle, where the first indication information indicates that the second route is abnormal.

After determining the included angle between the first route and the second route in the same reference system, the electronic device outputs the first indication information to the user when the included angle is greater than or equal to the preset angle. The first indication information may indicate that a movement direction is abnormal, and/or is used to correct a movement direction (or is understood as providing a correct movement direction for the user, for example, informing the user of an offset by which the user needs to move leftward or rightward).

The reference system mentioned above may be a virtual reference system, a plane (for example, a ground) reference system, a camera reference system of the electronic device, or the like. This is not specifically limited herein. For example, if the plane is the ground on which the crosswalk is located, the included angle between the first route and the second route in the same reference system may be understood as an included angle between a projection of the first route on the ground and the second route. In addition, the first route may be understood as a safe movement route of the user/the electronic device on the ground, and the second route may be understood as an actual movement route of the user/the electronic device on the ground.

Optionally, in a movement process of the user, the electronic device captures a plurality of frames of images of a front angle of view of the user. The included angle between the first route and the second route in the same reference system is greater than or equal to the preset angle in a preset time period. In this case, it may be understood that the included angle is always greater than or equal to the preset angle within the preset time period, and then the first indication information is output to the user. In this case, whether the movement direction of the user deviates from the crosswalk is determined by using the plurality of frames of images, to prevent misjudgment caused by a large deviation of one frame.

Optionally, the first indication information is output to the user when the area of the crosswalk in the plurality of frames of images may gradually decrease, and the included angle is greater than or equal to the preset angle. In other words, before outputting the first indication information to the user, the electronic device determines that the area of the crosswalk in the plurality of frames of images gradually decreases, to ensure that the user is standing at an intersection of the crosswalk and determine whether the user is really going to pass through the crosswalk.

The preset angle in this embodiment of this application may be set based on an actual requirement, for example, 45 degrees or 30 degrees. This is not specifically limited herein.

For example, an image in FIG. 11 is used as an example. The first indication information is output to the user if α is greater than or equal to the preset angle.

Optionally, if the object in the image includes the traffic light information, the length of the crosswalk, the motor vehicle information related to the crosswalk, and the like, the first indication information may further indicates a movement path of the user on the crosswalk, the traffic light information, an obstacle that affects movement of the user on the crosswalk, and the like. In this case, the user may determine the traffic light information, the movement path on the crosswalk, and/or the obstacle on the crosswalk based on the first indication information.

In this embodiment of this application, the first indication information includes at least one of the following representation forms, which are separately described below.

### 1. The first indication information is a voice.

When the first indication information is the voice, the user may determine, by listening to the first indication information, whether an abnormality occurs, and correct a movement direction.

For example, when the first indication information indicates that the movement direction is abnormal, the first indication information may be voice information "Please note that the movement direction is abnormal", or voice information "Please note that you have deviated from the crosswalk", or the like. When the first indication information is used to correct the movement direction, the first indication information may be voice information "You have deviated from the crosswalk. Please move to the right", or voice information "You have deviated from the crosswalk. Please move to the left", or the like. Certainly, the first indication information may alternatively be continuously broadcast to the user until an included angle between the actual movement route of the user and the safe movement route is less than a specific angle.

For example, the object in the image includes the traffic light information, the length of the crosswalk, the motor vehicle information related to the crosswalk, and the like. The first indication information may be "A current light is a red light, and the light turns to a green light in three seconds", or "You are currently on the crosswalk, and there is 2 meters left before passing through the crosswalk", or "There is a pedestrian at your front right side. Please pay attention", or the like.

In this case, in a scenario in which the user is a blind person, the blind person may determine, based on the voice, whether a movement direction of the blind person is abnormal, and/or how to correct the movement direction. In other words, the blind person can ensure safety based on the voice when the blind person passes through the crosswalk.

### 2. The first indication information is mark information presented on an image.

When the first indication information is the mark information, the user may determine, by looking down to view the first indication information, whether an abnormality occurs, and correct the movement direction.

For example, the first indication information may be mark information presented on an image. The user determines a deviation of the movement direction by viewing the mark information, and may correct an actual movement direction based on the deviation between the mark information and the actual movement direction, so that the user can safely pass through the crosswalk when looking down to view the electronic device.

This case is applicable to a scenario in which the user looks down to view the mobile phone to pass through the crosswalk or a scenario in which a deaf person passes through the crosswalk. Safety of the user who looks down or the deaf user when the user passes through the crosswalk can be ensured by viewing the mark information presented on the image.

For example, when the first indication information is the mark information, the first indication information may be as shown in FIG. 12.

It should be noted that the foregoing several cases may be used together. For example, the first indication information may include a voice and mark information, that is, the mark information is displayed to the user when the voice is played to the user. In addition, the foregoing several cases are merely examples. In actual application, the first indication information may be alternatively expressed in another form. This is not specifically limited herein.

In this embodiment of this application, the included angle between the safe walking route and the actual walking route of the user is determined, and when the included angle is greater than or equal to the preset angle, the first indication information is output to the user, to indicate that the actual walking direction of the user is abnormal. In this way, in a case in which the user deviates from the crosswalk in a movement process, safety of the user is improved by using the output first indication information when the user passes through the crosswalk.

Optionally, to ensure that the image obtained by the electronic device is more reasonable, before step 301, the pose of the electronic device may be further determined and/or adjusted.

In a possible implementation, a first pose in which the electronic device obtains the plurality of frames of images includes a first pitch angle and a first yaw angle, a value range of the first pitch angle is 60 to 100 degrees, and a value range of the first yaw angle is 60 to 120 degrees.

In another possible implementation, before obtaining the plurality of frames of images, the electronic device first obtains a second pose of the electronic device carried by the user. The electronic device further determines that a second pitch angle in the second pose is different from the first pitch angle, or a second yaw angle in the second pose is different from the first yaw angle. In other words, the second pose is not suitable for the electronic device to capture the image. In this case, second indication information may be output. That the second pose is not within a preset pose range may be understood as that the second pose is a pose that does not meet a photographing condition. The second indication information is used by the user to adjust the second pose of the electronic device to the first pose.

In this embodiment of this application, an azimuth at which when the electronic device captures the image is not limited.

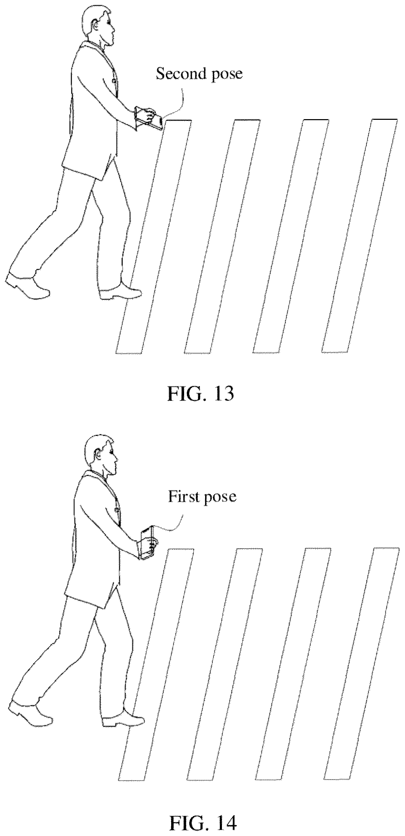

For example, the second pose of the electronic device is shown in FIG. 13. After the electronic device outputs the second indication information to the user, as shown in FIG. 14, the electronic device adjusts the second pose to the first pose.

Optionally, after step 303, third indication information is output to the user if a new image obtained by the electronic device does not include the crosswalk, where the third indication information indicates that the user has passed through the crosswalk.

The first indication information, the second indication information, and the third indication information in this embodiment of this application may be expressed in a form of voice, mark information, or the like. This is not specifically limited herein.

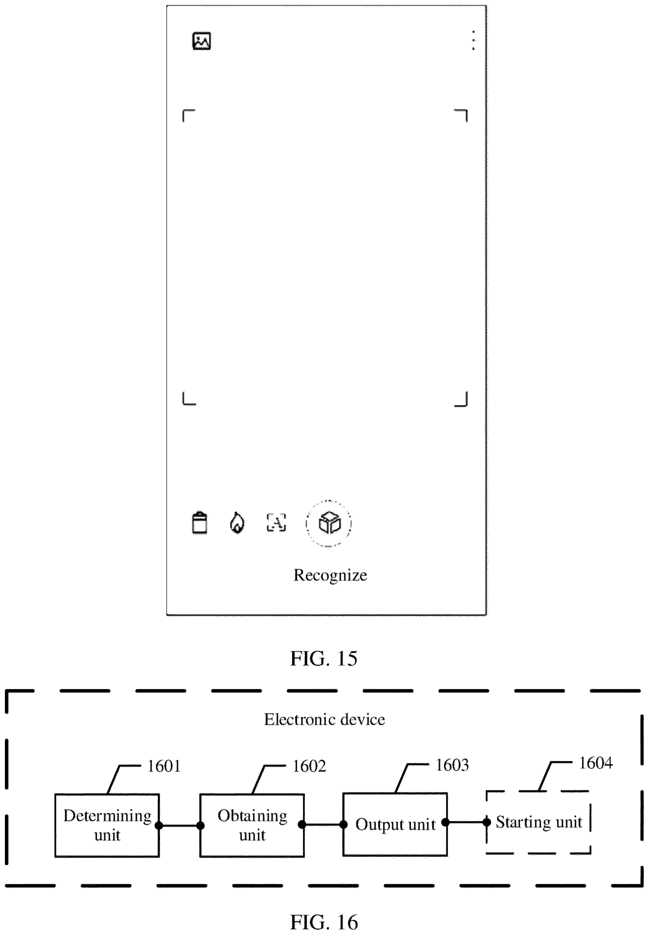

In addition, before step 301, the electronic device may start a first application based on an operation of the user, and the first application is used by the electronic device to obtain the plurality of frames of images of the front angle of view of the user. The function may also be referred to as smart recognition. The smart recognition may be an independent application, or may be a part of a new function of an existing application. For example, the smart recognition may be used as a new working mode in a camera application. The smart recognition may be started in a plurality of manners. For example, the smart recognition is started after an application is started and a smart recognition function is selected, the smart recognition is quickly started on a secondary page of a home screen, the smart recognition is started by using a voice assistant, or the smart recognition is quickly started by using a physical button. To facilitate use of a visually impaired user, the smart recognition may be started by using the voice assistant or the physical button. After the smart recognition is started, a smart recognition page is displayed. FIG. 15 shows an example of the smart recognition page.

The foregoing describes the image processing method in embodiments of this application, and the following describes an electronic device in embodiments of this application. Refer to FIG. 16. An embodiment of an electronic device in embodiments of this application includes:
a determining unit 1601, configured to determine a first route if a crosswalk is detected in a plurality of frames of images, where the first route is a safe walking route on the crosswalk;
an obtaining unit 1602, configured to obtain, based on the plurality of frames of images, a second route that a user actually walks; and
an output unit 1603, configured to output first indication information to the user if an included angle between the first route and the second route in a same reference system is greater than or equal to a preset angle, where the first indication information indicates that the second route is abnormal.

Optionally, the electronic device may further include a starting unit 1604, configured to start a first application based on an operation of the user, where the first application is used by the electronic device to obtain the plurality of frames of images of the front angle of view of the user.

In this embodiment, operations performed by the units in the electronic device are similar to those described in the embodiments shown in FIG. 1 to FIG. 14. Details are not described herein again.

In this embodiment, the included angle between the safe walking route and the actual walking route of the user is determined, and when the included angle is greater than or equal to the preset angle, the output unit 1603 outputs the first indication information to the user, to indicate that the actual walking direction of the user is abnormal. In this way, in a case in which the user deviates from the crosswalk in a movement process, safety of the user is improved by using the output first indication information when the user passes through the crosswalk.

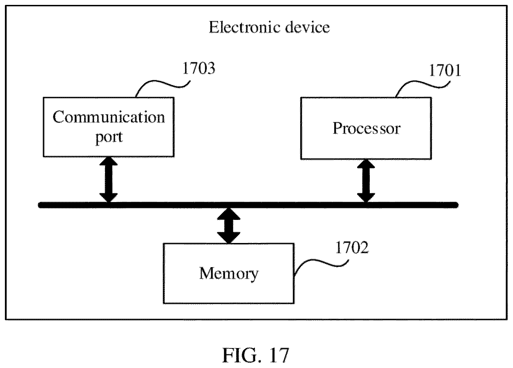

FIG. 17 is a schematic diagram of a structure of another electronic device according to an embodiment of this application. The electronic device may include a processor 1701, a memory 1702, and a communication port 1703. The processor 1701, the memory 1702, and the communication port 1703 are interconnected by using a line. The memory 1702 stores program instructions and data.

The memory 1702 stores program instructions and data that correspond to the steps performed by the electronic device in the corresponding implementations shown in FIG. 1 to FIG. 15.

The processor 1701 is configured to perform the steps performed by the electronic device in any one of the embodiments shown in FIG. 1 to FIG. 15.

The communication port 1703 may be configured to: receive and send data, and is configured to perform steps related to obtaining, sending, and receiving in any one of the embodiments shown in FIG. 1 to FIG. 15.

In an implementation, the electronic device may include more or fewer components than those shown in FIG. 17. This is merely an example for description, but is not limited in this application.

In the several embodiments provided in this application, it should be understood that the disclosed system, apparatus, and method may be implemented in other manners. For example, the described apparatus embodiment is merely an example. For example, division into the units is merely logical function division and may be other division in actual implementation. For example, a plurality of units or components may be combined or integrated into another system, or some features may be ignored or not performed. In addition, the displayed or discussed mutual couplings or direct couplings or communication connections may be implemented through some interfaces. The indirect couplings or communication connections between the apparatuses or units may be implemented in electrical, mechanical, or another form.

The units described as separate parts may or may not be physically separate, and parts displayed as units may or may not be physical units, may be located in one position, or may be distributed on a plurality of network units. Some or all of the units may be selected based on actual requirements to achieve the objectives of the solutions of embodiments.

In addition, function units in embodiments of this application may be integrated into one processing unit, each of the units may exist alone physically, or two or more units are integrated into one unit. All or some of the foregoing integrated units may be implemented by using software, hardware, firmware, or any combination thereof.

When the software is used to implement the integrated units, all or a part of the integrated units may be implemented in a form of a computer program product. The computer program product includes one or more computer instructions. When the computer program instructions are loaded and executed on the computer, the procedure or functions according to embodiments of the present invention are all or partially generated. The computer may be a general-purpose computer, a dedicated computer, a computer network, or other programmable apparatuses. The computer instructions may be stored in a computer-readable storage medium or may be transmitted from a computer-readable storage medium to another computer-readable storage medium. For example, the computer instructions may be transmitted from a website, computer, server, or data center to another website, computer, server, or data center in a wired (for example, a coaxial cable, an optical fiber, or a digital subscriber line (digital subscriber line, DSL)) or wireless (for example, infrared, radio, or microwave) manner. The computer-readable storage medium may be any usable medium accessible by the computer, or a data storage device, for example, a server or a data center, integrating one or more usable media. The usable medium may be a magnetic medium (for example, a floppy disk, a hard disk, or a magnetic tape), an optical medium (for example, a DVD), a semiconductor medium (for example, a solid-state drive (solid-state drive, SSD)), or the like.

In the specification, claims, and accompanying drawings of this application, the terms "first", "second", and so on are intended to distinguish between similar objects but do not necessarily indicate a specific order or sequence. It should be understood that the terms used in such a way are interchangeable in proper circumstances, which is merely a discrimination manner that is used when objects having a same attribute are described in embodiments of this application. In addition, the terms "include", "contain" and any other variants mean to cover the non-exclusive inclusion, so that a process, method, system, product, or device that includes a series of units is not necessarily limited to those units, but may include other units not expressly listed or inherent to such a process, method, system, product, or device.

In the specification, claims, and accompanying drawings of this application, "at least one" means one or more, and "a plurality of" means two or more. The term "and/or" describes an association relationship for describing associated objects and represents that three relationships may exist. For example, A and/or B may indicate a case in which only A exists, both A and B exist, or only B exists, where A and B may be singular or plural. The character "/" generally indicates an "or" relationship between the associated objects. "At least one item (piece) of the following" or a similar expression thereof means any combination of these items, including a singular item (piece) or any combination of plural items (pieces). For example, at least one of a, b, or c may indicate: a; b; c; a and b; a and c; b and c; or a, b, and c; where a, b, and c may be singular or plural.

## Claims

1. An image processing method, wherein the method is applied to an electronic device, the electronic device is configured to obtain a plurality of frames of images of a front angle of view of a user, and the method comprises:
determining a first route if a crosswalk is detected in the plurality of frames of images, wherein the first route is a safe walking route on the crosswalk;
obtaining, based on the plurality of frames of images, a second route that the user actually walks; and
outputting first indication information to the user if an included angle between the first route and the second route in a same reference system is greater than or equal to a preset angle, wherein the first indication information indicates that the second route is abnormal.

2. The method according to claim 1, wherein a first pose in which the electronic device obtains the plurality of frames of images comprises a first pitch angle and a first yaw angle, a value range of the first pitch angle is 60 degrees to 100 degrees, and a value range of the first yaw angle is 60 degrees to 120 degrees.

3. The method according to claim 2, wherein the method further comprises:
obtaining a second pose of the electronic device, wherein a second pitch angle in the second pose is different from the first pitch angle, or a second yaw angle in the second pose is different from the first yaw angle; and
outputting second indication information, wherein the second indication information is used for the user to adjust the second pose to the first pose.

4. The method according to claim 2 or 3, wherein the first route is an angular bisector in which an included angle formed by extension directions of two edges of the crosswalk is located, or the first route is parallel to the edge of the crosswalk.

5. The method according to any one of claims 1 to 4, wherein before the outputting first indication information to the user, the method further comprises:
determining that an area of the crosswalk in the plurality of frames of images gradually decreases.

6. The method according to any one of claims 1 to 5, wherein a movement route in which the electronic device captures the plurality of frames of images is the second route, and the movement route is parallel to a ground on which the crosswalk is located.

7. The method according to any one of claims 1 to 6, wherein after the outputting first indication information to the user, the method further comprises:
outputting third indication information to the user if a new image obtained by the electronic device does not comprise the crosswalk, wherein the third indication information indicates that the user has passed through the crosswalk.

8. The method according to any one of claims 1 to 7, wherein the plurality of frames of images further comprise traffic light information, a length of the crosswalk, and motor vehicle information related to the crosswalk; the traffic light information comprises a color and duration of a traffic light, and the motor vehicle information comprises a traveling direction and a distance of a motor vehicle relative to the crosswalk; and the first indication information further indicates a movement path of the user on the crosswalk.

9. The method according to any one of claims 1 to 8, wherein before the determining a first route, the method further comprises:
starting a first application based on an operation of the user, wherein the first application is used by the electronic device to obtain the plurality of frames of images of the front angle of view of the user.

10. The method according to any one of claims 1 to 9, wherein a representation form of the first indication information comprises a voice and/or mark information.

11. An electronic device, wherein the electronic device is configured to obtain a plurality of frames of images of a front angle of view of a user, and the electronic device comprises:
a determining unit, configured to determine a first route if a crosswalk is detected in the plurality of frames of images, wherein the first route is a safe walking route on the crosswalk;
an obtaining unit, configured to obtain, based on the plurality of frames of images, a second route that the user actually walks; and
an output unit, configured to output first indication information to the user if an included angle between the first route and the second route in a same reference system is greater than or equal to a preset angle, wherein the first indication information indicates that the second route is abnormal.

12. The electronic device according to claim 11, wherein a first pose in which the electronic device obtains the plurality of frames of images comprises a first pitch angle and a first yaw angle, a value range of the first pitch angle is 60 degrees to 100 degrees, and a value range of the first yaw angle is 60 degrees to 120 degrees.

13. The electronic device according to claim 12, wherein the obtaining unit is further configured to obtain a second pose of the electronic device, wherein a second pitch angle in the second pose is different from the first pitch angle, or a second inclusion angle in the second pose is different from the first inclusion angle; and
the output unit is further configured to output second indication information, wherein the second indication information is used for the user to adjust the second pose to the first pose.

14. The electronic device according to claim 12 or 13, wherein the first route is an angular bisector in which an included angle formed by extension directions of two edges of the crosswalk is located, or the first route is parallel to the edge of the crosswalk.

15. The electronic device according to any one of claims 11 to 14, wherein the determining unit is further configured to determine that an area of the crosswalk in the plurality of frames of images gradually decreases.

16. The electronic device according to any one of claims 11 to 15, wherein a movement route in which the electronic device captures the plurality of frames of images is the second route, and the movement route is parallel to a ground on which the crosswalk is located.

17. The electronic device according to any one of claims 11 to 16, wherein the output unit is further configured to output third indication information to the user if a new image obtained by the electronic device does not comprise the crosswalk, wherein the third indication information indicates that the user has passed through the crosswalk.

18. The electronic device according to any one of claims 11 to 17, wherein the plurality of frames of images further comprise traffic light information, a length of the crosswalk, and motor vehicle information related to the crosswalk; the traffic light information comprises a color and duration of a traffic light, and the motor vehicle information comprises a traveling direction and a distance of a motor vehicle relative to the crosswalk; and the first indication information further indicates a movement path of the user on the crosswalk.

19. The electronic device according to any one of claims 11 to 18, wherein the electronic device further comprises:
a starting unit, configured to start a first application based on an operation of the user, wherein the first application is used by the electronic device to obtain the plurality of frames of images of the front angle of view of the user.

20. The electronic device according to any one of claims 11 to 19, wherein a representation form of the first indication information comprises a voice and/or mark information.

21. An electronic device, comprising a processor, wherein the processor is coupled to a memory, the memory is configured to store a program or instructions, and when the program or the instructions is/are executed by the processor, the electronic device is enabled to perform the method according to any one of claims 1 to 10.

22. A computer storage medium, comprising computer instructions, wherein when the computer instructions are run on a terminal device, the terminal device is enabled to perform the method according to any one of claims 1 to 10.

23. A computer program product, wherein when the computer program product runs on a computer, the computer is enabled to perform the method according to any one of claims 1 to 10.
